# EUROPÄISCHE PATENTSCHRIFT

(11) **EP 4 106 840 B1**
(45) Veröffentlichungstag und Bekanntmachung des Hinweises auf die Patenterteilung: **03.07.2024**
(21) Anmeldenummer: 22700088.2
(22) Anmeldetag: 11.01.2022
(51) Int. Cl.: A61M 5/145, A61M 5/50

(54) **VORRICHTUNGEN ZUR VERABREICHUNG VON MEDIZINISCHER FLÜSSIGKEIT SOWIE ENTSPRECHENDE VERFAHREN**
DEVICES FOR ADMINISTERING A MEDICAL FLUID AND CORRESPONDING METHODS
DISPOSITIFS POUR L'ADMINISTRATION DE LIQUIDES MÉDICAUX ET PROCÉDÉS CORRESPONDANTS

(30) Priorität: 15.01.2021 DE 102021100818
(43) Veröffentlichungstag der Anmeldung: 28.12.2022
(73) Patentinhaber: B. Braun Melsungen AG, 34212 Melsungen (DE)
(72) Erfinder: BÜRGER, Maria, 34323 Malsfeld (DE); ERLEN, Christoph, 34132 Kassel (DE); ROEPER, Jana, 34119 Kassel (DE)
(74) Vertreter: Winter, Brandl - Partnerschaft mbB
(86) Internationale Anmeldenummer: PCT/EP2022/050376
(87) Internationale Veröffentlichungsnummer: WO 2022/152663

(56) Entgegenhaltungen:
- US-A1- 2008 312 512
- US-A1- 2014 100 526
- US-A1- 2017 106 139
- US-A1- 2019 096 518
- US-A1- 2019 099 559
- US-A1- 2019 351 138
- US-B1- 7 267 666

## Beschreibung

### Technisches Gebiet

Die vorliegende Offenbarung bezieht sich auf eine Vorrichtung zur Verabreichung von medizinischer Flüssigkeit gemäß dem Oberbegriff des Anspruchs 1 beziehungsweise des Anspruchs 6, insbesondere auf eine Spritzen- oder Infusionspumpe mit einer Abdeckung, die mit einer manuell sperrbaren Eingabeeinheit ausgestattet ist und die geeignet ist, in einer geschlossenen Stellung eine in einem Aufnahmebereich der Spritzenpumpe aufgenommene Spritze beziehungsweise einen in einem Aufnahmebereich der Infusionspumpe aufgenommen Schlauch zumindest abschnittsweise abzudecken, und mit einem Antrieb, der geeignet ist, auf eine in der Spritzenpumpe aufgenommene Spritze beziehungsweise auf einen in der Infusionspumpe aufgenommenen Schlauch derart einzuwirken, dass in der aufgenommenen Spritze beziehungsweise in dem aufgenommenen Schlauch enthaltene Flüssigkeit gefördert wird. Des Weiteren betrifft die vorliegende Offenbarung Verfahren zum Betrieb von Vorrichtungen zur Verabreichung von medizinischer Flüssigkeit gemäß dem Oberbegriff des Anspruchs 14 beziehungsweise des Anspruchs 15.

### Stand der Technik

Eine gattungsgemäße Vorrichtung zum Verabreichen von medizinischer Flüssigkeit wird beispielsweise in CN 211751505 U offenbart. Die Spritzenpumpe gemäß CN 211751505 U weist einen Hauptkörper auf, der an einer in Gebrauchslage vorderen Seite mit einem Aufnahmebereich zur Aufnahme einer Spritze ausgestattet ist. Des Weiteren weist die Vorrichtung eine Abdeckung auf, die mit dem Hauptkörper schwenkbar verbunden ist und die in einer geschlossenen Stellung den Aufnahmebereich abdeckt. An der Abdeckung ist ein berührungsempfindlicher Bildschirm vorgesehen, der sich in Gebrauchslage der Vorrichtung an einer vorderen Seite erstreckt, wenn sich die Abdeckung in ihrer geschlossenen Stellung befindet. Die Vorrichtung weist eine Steuereinheit auf. Mittels eines Schlüssels kann die Steuereinheit dazu veranlasst werden, den Bildschirm und die Abdeckung zu entsperren.

Weitere Vorrichtungen mit sperrbaren Eingabeeinheiten werden zum Beispiel in US 2017 / 0 106 139 A1 und US 2008 / 0 312 512 A1 offenbart.

US 2019 / 0 096 518 A1, US 2014 / 0 100 526 A1, US 2019 / 0 099 559 A1, US 2019 / 0 351 138 A1 und US 7 267 666 B1 offenbaren Infusionspumpen mit Eingabeeinheiten, die nicht dazu ausgebildet sind, gesperrt zu werden.

Problem der Vorrichtung gemäß CN 211751505 U ist, dass zu ihrem sicheren Betrieb ein Schlüssel notwendig ist.

Aufgabe der vorliegenden Erfindung ist es deshalb, eine Vorrichtung zur Verabreichung von medizinischer Flüssigkeit bereitzustellen, deren Betrieb einfacher ausgestaltet sein kann.

Diese Aufgabe wird durch Merkmale der Ansprüche 1, 6, 14 und 15 gelöst. Vorteilhafte Weiterbildungen sind Gegenstand der Unteransprüche.

Eine offenbarungsgemäße Vorrichtung zur Verabreichung von medizinischer Flüssigkeit ist insbesondere eine Spritzenpumpe oder eine Infusionspumpe. Die Vorrichtung weist einen Hauptkörper auf. Der Hauptkörper weist einen Aufnahmebereich und einen Antrieb auf. Der Aufnahmebereich ist geeignet, einen flüssigkeitsbeinhaltenden Hohlkörper zumindest abschnittsweise aufzunehmen. Ein Hohlkörper im Sinne der vorliegenden Offenbarung kann insbesondere eine Spritze, ein Infusionsschlauch oder ein sonstiger Schlauch eines medizinischen Systems sein. Der Antrieb ist geeignet, auf einen zumindest abschnittsweise aufgenommenen Hohlkörper derart einzuwirken, dass eine in dem zumindest abschnittsweise aufgenommenen Hohlkörper beinhaltete Flüssigkeit gefördert wird. Insbesondere ist der Antrieb in der Lage, einen Kolben einer aufgenommenen Spritze zu bewegen oder mittels einer Peristaltik auf einen Abschnitt eines aufgenommenen Schlauchs einzuwirken.

Zur Steuerung des Antriebs weist die Vorrichtung eine Steuereinheit auf.

Die Vorrichtung weist eine Abdeckung auf, die mit dem Hauptkörper beweglich, insbesondere schwenkbar, verbundenen ist. Insbesondere erstreckt sich eine Schwenkachse der Abdeckung in Gebrauchslage der Vorrichtung vorne in einem Bereich einer Unterkante eines Gehäuses der Vorrichtung. Die Abdeckung deckt in einer geschlossenen Stellung den Aufnahmebereich nach außen hin ab.

"Abdecken" im Sinne der vorliegenden Offenbarung bedeutet, dass ein im Aufnahmebereich der Vorrichtung zumindest abschnittsweise aufgenommener Hohlkörper nicht ohne Bewegen bzw. Verschwenken der Abdeckung aus der Vorrichtung, zumindest nicht ohne Beschädigungen, entnommen werden kann.

An der Abdeckung ist eine manuell sperrbare Eingabeeinheit vorgesehen. Über die Eingabeeinheit können Befehle an die Steuereinheit eingegeben werden.

Die Eingabeeinheit kann insbesondere in Form eines manuell sperrbaren berührungsempfindlichen Bildschirms (auch unter der Bezeichnung Touchscreen oder Touchdisplay bekannt) ausgebildet sein. Alternativ kann die Eingabeeinheit auch in Form von Tasten ausgebildet sein oder die Eingabeeinheit kann zusätzlich zu einem manuell sperrbaren berührungsempfindlichen Bildschirm Tasten aufweisen.

"Sperrbar" im Zusammenhang mit der Eingabeeinheit bedeutet im Rahmen der Offenbarung, dass die Eingabeeinheit in einen gesperrten Zustand gebracht werden kann, in welchem der Umfang der möglichen Eingaben eingeschränkt ist. Insbesondere kann der gesperrte Zustand derart konfiguriert sein, dass lediglich eine Entsperrungsbedienungssequenz eingegeben werden kann. Als "Bedienungssequenz" wird im Sinne der vorliegenden Offenbarung eine Abfolge mindestens zweier Eingabe verstanden. Um die Eingabeeinheit manuell zu sperren, kann eine Sperrungsbedienungssequenz vorgesehen sein. Um die Eingabeeinheit zu entsperren, kann die genannte Entsperrungsbedienungssequenz vorgesehen sein.

Falls die Eingabeeinheit als berührungsempfindlicher Bildschirm ausgebildet ist, kann die Entsperrungsbedienungssequenz derart ausgestaltet sein, dass der Bildschirm in einem ersten Schritt berührt werden muss, um eine Anzeige eines Hinweises auszulösen, und in einem zweiten Schritt in diesem Hinweis eine entsprechende Fläche gedrückt werden muss, um den Bildschirm zu entsperren.

Alternativ beziehungsweise zusätzlich kann die Vorrichtung derart ausgebildet sein, dass ein Entsperren der Eingabeeinheit mittels eines separat an dem Hauptkörper oder an der Abdeckung angebrachten beispielsweise elektromechanischen oder kapazitiven Taster oder mittels mehrerer separat an dem Hauptkörper und/oder an der Abdeckung angebrachten beispielsweise elektromechanischen oder kapazitiven Taster bewerkstelligt werden kann.

Die Eingabeeinheit ist an der Abdeckung derart vorgesehen, dass es in der geschlossenen Stellung der Abdeckung möglich ist, von außen zumindest auf einen Abschnitt der Eingabeeinheit zuzugreifen, um eine manuelle Eingabe auf der Eingabeeinheit vorzunehmen. Insbesondere ist die Eingabeeinheit an der Abdeckung derart vorgesehen, dass sie sich in der geschlossenen Stellung der Abdeckung gänzlich an einer Außenseite der Vorrichtung, insbesondere an einer in Gebrauchslage der Vorrichtung vorderen Außenseite, erstreckt.

Die Vorrichtung weist einen Sensor auf, der geeignet ist, zu erkennen, ob die Abdeckung in der geschlossenen Stellung ist. Der Sensor kann als Taster ausgebildet sein, der auf eine durch ein Bewegen der Abdeckung verursachte Berührung anspricht. Der Sensor kann als Lagesensor ausgebildet sein, der die Stellung eines mit der Abdeckung und dem Hauptkörper verbundenen elektromechanischen Schalters und somit die relative Lage der Abdeckung zum Hauptkörper detektieren kann. Der Sensor kann als Hallsensor ausgebildet sein, der auf ein Bewegen eines in der Abdeckung vorgesehenen Magneten anspricht. Der Sensor kann im Rahmen einer Lichtschranke als lichtempfindliches Element ausgebildet sein.

Die Steuereinheit ist ausgebildet ist, bei einem von dem Sensor erkannten Öffnen der Abdeckung zu ermitteln, ob die Eingabeeinheit gesperrt ist, und, falls die Eingabeeinheit bei dem erkannten Öffnen der Abdeckung nicht gesperrt ist, die Eingabeeinheit zu sperren.

Der Vorteil der Vorrichtung gemäß der vorliegenden Offenbarung ist, dass eine Sperrung der Eingabeeinheit bei einem Öffnen der Abdeckung automatisch gewährleistet werden kann.

Gemäß einem Aspekt der Offenbarung kann die Steuereinheit ausgebildet sein, nach einem Öffnen der Abdeckung und einem dadurch verursachten Sperren der Eingabeeinheit bei einem von dem Sensor erkannten Schließen der Abdeckung die Eingabeeinheit zu entsperren. Somit ist es möglich, nach einem zeitweisen Öffnen der Abdeckung, das heißt, unmittelbar nach dem Schließen der Abdeckung manuelle Eingaben auf der Eingabeeinheit zu tätigen, ohne zuvor die Eingabeeinheit manuell, beispielweise mithilfe eines Schlüssels, zu entsperren.

Gemäß einem Aspekt der Offenbarung kann die Steuereinheit ausgebildet sein, nach einer Sperrung der Eingabeeinheit, die nicht durch ein Öffnen der Abdeckung verursacht wurde, und nach einem Öffnen der Abdeckung bei einem von dem Sensor erkannten Schließen der Abdeckung die Sperrung der Eingabeeinheit, insbesondere gegen ungewollte Eingaben, aufrechtzuerhalten. Anders ausgedrückt kann eine Priorisierung einer nicht durch ein Öffnen verursachten Sperrung, beispielsweise einer durch manuelles Eingeben veranlasste Sperrung, vorgenommen werden. Wie nachfolgend beschrieben, kann die Vorrichtung derart ausgebildet sein, dass eine Sperrung der Eingabeeinheit nicht nur durch das Öffnen der Abdeckung oder durch manuelles Eingeben der Sperrungsbedienungssequenz, sondern auch automatisch nach Ablauf einer vorbestimmten Zeit ohne Eingaben auf der Eingabeeinheit oder durch eine übermäßige Lageänderung der Vorrichtung ausgelöst wird. Gemäß dem vorliegenden Aspekt der Offenbarung kann eine dieser anderen Arten der Sperrung der Eingabeeinheit derart bevorzugt werden, dass ein automatisches Entsperren beim Schließen der Abdeckung nicht vollzogen wird. Dabei ist es unerheblich, wann die nicht durch das Öffnen der Abdeckung verursachte Sperrung erfolgt. Beispielsweise kann die Eingabeeinheit nach dem Öffnen und einem damit verbundenen automatischen Sperren der Eingabeeinheit zunächst manuell, beispielsweise durch Eingeben der Entsperrungsbedienungssequenz, entsperrt werden und dann, beispielsweise durch Unterlassen weiterer Eingaben über eine vorbestimmte Zeitspanne automatisch gesperrt werden. Wird die Abdeckung dann wieder geschlossen, bleibt die Eingabeeinheit gemäß dem vorliegenden Aspekt weiter gesperrt. Auch wenn bereits vor einem Öffnen der Abdeckung die Eingabeeinheit beispielsweise mittels Eingeben der Sperrungsbedienungssequenz, aufgrund längerer Inaktivität oder wegen einer übermäßigen Lageänderung der Vorrichtung gesperrt wird, bewirkt ein nach dem Öffnen der Abdeckung erfolgendes Schließen der Abdeckung gemäß dem vorliegenden Aspekt der Offenbarung keine Entsperrung der Eingabeeinheit. Somit ist es möglich, ein unbeabsichtigtes Entsperren der Eingabeeinheit zu verhindern.

Gemäß einem Aspekt der Offenbarung kann die Steuereinheit ausgebildet sein, eine vor einem Öffnen der Abdeckung begonnene und bei dem Öffnen der Abdeckung andauernde manuelle Eingabe aufzuheben beziehungsweise nicht zu berücksichtigen beziehungsweise rückgängig zu machen. Somit ist es möglich, bei einem manuellen Öffnen der Abdeckung ohne Rücksicht auf etwaige versehentliche Eingaben auf der Eingabeeinheit, die Abdeckung zu ergreifen. Hierbei ist der Unterschied zwischen einer einzelnen Eingabe und einer Eingabesequenz zu beachten. Die offenbarungsgemäße Vorrichtung kann derart ausgebildet sein, dass sich das Zusammenspiel eines Eingebens einer Eingabesequenz und einer Sperrung der Eingabeeinheit wie folgt darstellt. Wird eine aus mehreren einzelnen Eingaben bestehende Eingabesequenz durch Eingeben einer ersten Eingabe begonnen und wird die Abdeckung geöffnet, verhindert die Sperrung der Eingabeeinheit zunächst das Eingeben der übrigen Eingaben der Eingabesequenz. Wird die Eingabeeinheit dann beispielsweise durch Schließen der Abdeckung wieder entsperrt, kann mit dem Eingeben der Eingabesequenz fortgefahren werden ohne die bereits eingegebene Eingabe wiederholen zu müssen. Der vorliegende Aspekt der Offenbarung betrifft demgegenüber den Fall, dass die Abdeckung während des Eingebens einer einzelnen Eingabe, beispielsweise während eines Drückens einer Taste der Eingabeeinheit, geöffnet wird. Ein solches Öffnen der Abdeckung und die dadurch ausgelöste Sperrung der Eingabeeinheit während des Eingebens einer einzelnen Eingabe führt gemäß dem vorliegenden Aspekt der Offenbarung dazu, dass die angefangene aber noch nicht vollendete einzelne Eingabe verworfen wird.

Gemäß einem Aspekt der Offenbarung kann die Vorrichtung einen Sensor aufweisen, der geeignet ist, zu erkennen, ob ein Hohlkörper in dem Aufnahmebereich aufgenommen ist, und kann die Steuereinheit ausgebildet sein, ein von der Stellung der Abdeckung abhängiges Entsperren und/oder Sperren der Eingabeeinheit nur bei Erkennung eines in dem Aufnahmebereich aufgenommenen Hohlkörpers durchzuführen. Somit kann die Effizienz der Vorrichtung gesteigert werden.

Gemäß einem alternativen Aspekt betrifft die vorliegende Offenbarung eine Vorrichtung zur Verabreichung von medizinischer Flüssigkeit mit einer verriegelbaren Abdeckung. Insbesondere kann die Vorrichtung eine Spritzen- oder Infusionspumpe sein.

Die Vorrichtung weist einen Hauptkörper mit einem Aufnahmebereich und einem Antrieb auf. Der Aufnahmebereich ist geeignet, einen flüssigkeitsbeinhaltenden Hohlkörper zumindest abschnittsweise aufzunehmen. Der Hohlkörper kann eine Spritze, ein Infusionsschlauch oder ein sonstiger Schlauch eines medizinischen Systems sein. Der Antrieb ist geeignet, auf einen zumindest abschnittsweise aufgenommenen Hohlkörper derart einzuwirken, dass eine in dem zumindest abschnittsweise aufgenommenen Hohlkörper beinhaltete Flüssigkeit gefördert wird. Insbesondere ist der Antrieb in der Lage, einen Kolben einer aufgenommenen Spritze zu bewegen oder mittels einer Peristaltik auf einen Abschnitt eines aufgenommenen Schlauchs einzuwirken.

Die Vorrichtung weist eine Steuereinheit zur Steuerung des Antriebs auf.

Des Weiteren weist die Vorrichtung eine mit dem Hauptkörper beweglich, insbesondere schwenkbar, verbundene Abdeckung auf. Insbesondere erstreckt sich eine Schwenkachse der Abdeckung in Gebrauchslage der Vorrichtung vorne in einem Bereich einer Unterkante eines Gehäuses der Vorrichtung. Die Abdeckung deckt in einer geschlossenen Stellung den Aufnahmebereich nach außen hin ab.

An der Abdeckung ist eine manuell sperrbare Eingabeeinheit vorgesehen. Über die Eingabeeinheit können Befehlen an die Steuereinheit eingegeben werden.

Die Eingabeeinheit kann insbesondere in Form eines manuell sperrbaren berührungsempfindlichen Bildschirms (auch unter der Bezeichnung Touchscreen oder Touchdisplay bekannt) ausgebildet sein. Alternativ kann die Eingabeeinheit auch in Form von Tasten ausgebildet sein oder die Eingabeeinheit kann zusätzlich zu einem manuell sperrbaren berührungsempfindlichen Bildschirm Tasten aufweisen. Auch im Übrigen treffen die voranstehenden Aussagen bezüglich der Definitionen und Spezifikationen der obligatorischen und fakultativen Merkmale der Eingabeeinheit der Vorrichtung mit der nicht-verriegelbaren Abdeckung auch auf die Eingabeeinheit der Vorrichtung mit verriegelbarer Abdeckung zu.

Die Eingabeeinheit ist an der Abdeckung derart vorgesehen, dass es in der geschlossenen Stellung der Abdeckung möglich ist, von außen zumindest auf einen Abschnitt der Eingabeeinheit zuzugreifen, um eine manuelle Eingabe auf der Eingabeeinheit vorzunehmen. Insbesondere ist die Eingabeeinheit an der Abdeckung derart vorgesehen, dass sie sich in der geschlossenen Stellung der Abdeckung gänzlich an einer Außenseite der Vorrichtung, insbesondere an einer in Gebrauchslage der Vorrichtung vorderen Außenseite, erstreckt.

Die Vorrichtung gemäß dem alternativen Aspekt weist eine Verriegelungsvorrichtung auf, die geeignet ist, die Abdeckung in der geschlossenen Stellung zu verriegeln.

Des Weiteren weist die Vorrichtung eine Sensoreinheit auf, die geeignet ist, zu erkennen, ob die Abdeckung in der geschlossenen Stellung verriegelt ist. Dazu kann die Sensoreinheit zwei Sensoren aufweisen, von welchen einer die Stellung der Abdeckung relativ zu dem Hauptkörper ermittelt und der andere die Stellung der Verriegelungsvorrichtung ermittelt. Der Sensor bezüglich der Abdeckung kann, wie voranstehend beschrieben, als Taster ausgebildet sein, der auf eine durch ein Bewegen der Abdeckung verursachte Berührung anspricht oder kann als Lagesensor ausgebildet sein, der die Stellung eines mit der Abdeckung und dem Hauptkörper verbundenen elektromechanischen Schalters und somit die relative Lage der Abdeckung zum Hauptkörper detektieren kann, oder kann als Hallsensor ausgebildet sein, der auf ein Bewegen eines in der Abdeckung vorgesehenen Magneten anspricht, oder kann im Rahmen einer Lichtschranke als lichtempfindliches Element ausgebildet sein. Der Sensor bezüglich der Stellung der Verriegelungsvorrichtung kann als Taster ausgebildet sein, der auf eine von einer Bewegung in der Verriegelungsvorrichtung verursachte Berührung anspricht. Der Sensor bezüglich der Verriegelungsvorrichtung kann als Hallsensor ausgebildet sein, der auf ein Bewegen eines in der Verriegelungsvorrichtung, insbesondere in oder an einem beweglichen Riegel, vorgesehenen Magneten anspricht. Der Sensor bezüglich der Verriegelungsvorrichtung kann mittels eines Potentiometers umgesetzt sein, das mit einem beweglichen Riegel verbunden ist. Der Sensor bezüglich der Verriegelungsvorrichtung kann im Rahmen einer Lichtschranke als lichtempfindliches Element ausgebildet sein.

Die Sensoreinheit kann auch mithilfe nur eines Sensors umgesetzt sein, welcher die Relativposition des mit dem Hauptkörper verbundenen Riegels bezüglich der Abdeckung ermittelt, so dass die unterschiedlichen Zustände "geschlossen und verriegelt", "geschlossen und entriegelt", und "geöffnet und entriegelt" lediglich anhand eines Signals ermittelt werden. Dies kann beispielsweise dadurch umgesetzt sein, dass in der Abdeckung ein Hallsensor und in oder an dem Riegel der Verriegelungsvorrichtung ein Magnet vorgesehen sind.

Die Steuereinheit ist ausgebildet, bei einem von der Sensoreinheit erkannten Entriegeln der geschlossenen Abdeckung zu ermitteln, ob die Eingabeeinheit gesperrt ist, und, falls die Eingabeeinheit bei dem erkannten Entriegeln der Abdeckung nicht gesperrt ist, die Eingabeeinheit zu sperren. Insbesondere kann die Steuereinheit bzw. die Vorrichtung derart ausgebildet sein, dass ein, insbesondere mittels manueller Eingabe über die Eingabeeinheit, erfolgter Befehl zum Entriegeln der Abdeckung in der geschlossenen Stellung auch ein automatisches Öffnen der Abdeckung verursacht.

Die Vorrichtung gemäß dem alternativen Aspekt der vorliegenden Offenbarung sichert in vorteilhafter Weise die Eingabeeinheit vor versehentlichen Eingaben.

Gemäß einem Aspekt der Offenbarung kann die Steuereinheit ausgebildet sein, nach einem Entriegeln und Öffnen der Abdeckung und einem durch das Entriegeln verursachten Sperren der Eingabeeinheit bei einem von der Sensoreinheit erkannten Schließen und Verriegeln der Abdeckung die Eingabeeinheit zu entsperren. Somit kann nach einem Verriegeln einer zuvor entriegelten Abdeckung ohne zusätzliche weitere Schritte oder Maßnahmen vonseiten eines Bedieners eine manuelle Eingabe auf der entsperrten Eingabeeinheit erfolgen.

Gemäß einem Aspekt der Offenbarung kann die Steuereinheit ausgebildet sein, nach einer Sperrung der Eingabeeinheit, die nicht durch ein Entriegeln der Abdeckung verursacht wurde und nach einem Entriegeln der Abdeckung bei einem von der Sensoreinheit erkannten Verriegeln der Abdeckung die Sperrung der Eingabeeinheit aufrechtzuerhalten. Somit ist es möglich, die Sicherheit der Vorrichtung zu verbessern. Der Begriff "nicht durch Entriegeln der Abdeckung verursachte Sperrung der Eingabeeinheit" bezieht sich zum Beispiel auf ein manuelles Sperren mittels Eingeben einer Sperrungsbedienungssequenz, ein automatisches Sperren nach Ablauf einer vorbestimmten Zeit ohne Eingaben auf der Eingabeeinheit oder auf ein automatisches Sperren aufgrund einer Lageänderung der Vorrichtung. Gemäß dem vorliegenden Aspekt der Offenbarung ist es unerheblich, ob das nicht durch Entriegeln der Abdeckung verursachte Sperren der Eingabeeinheit vor oder nach dem Entriegeln der Abdeckung erfolgt.

Gemäß einem Aspekt der Offenbarung kann die Steuereinheit ausgebildet sein, eine vor einem Entriegeln der Abdeckung begonnene und bei dem Entriegeln der Abdeckung andauernde manuelle Eingabe aufzuheben. Somit ist es möglich, bei einem manuellen Entriegeln der Abdeckung die Abdeckung zu ergreifen, ohne ein Eingeben eines nicht beabsichtigten Befehls befürchten zu müssen. Hierbei ist der bereits erwähnte Unterschied zwischen einzelnen Eingaben und aus einzelnen Eingaben bestehenden Eingabesequenzen zu beachten.

Gemäß einem Aspekt der Offenbarung kann die Vorrichtung einen Sensor aufweisen, der geeignet ist, zu erkennen, ob ein Hohlkörper in dem Aufnahmebereich aufgenommen ist, und kann die Steuereinheit ausgebildet sein, ein von der Verriegelung der geschlossenen Abdeckung abhängiges Entsperren und/oder Sperren der Eingabeeinheit nur bei Erkennung eines in dem Aufnahmebereich aufgenommenen Hohlkörpers durchzuführen. Somit ist es möglich, die Effizienz der Vorrichtung zu steigern.

Gemäß einem Aspekt der Offenbarung kann die Steuereinheit ausgebildet sein, die Eingabeeinheit nach einer Entsperrung und eines darauffolgenden Ablaufs eines Zeitintervalls, in dem keine manuelle Eingabe auf der Eingabeeinheit erfolgt, zu sperren. Somit ist es möglich, die Sicherheit der Vorrichtung weiter zu verbessern.

Gemäß einem Aspekt der Offenbarung kann die Vorrichtung einen Lagesensor aufweisen, der geeignet ist, eine Lageänderung des Hauptkörpers zu erkennen, und kann die Steuereinheit ausgebildet sein, bei einem Überschreiten der erkannten Lageänderung über einen vorbestimmten Schwellenwert zu ermitteln, ob die Eingabeeinheit gesperrt ist, und, falls die Eingabeeinheit nicht gesperrt ist, die Eingabeeinheit zu sperren. Somit kann ein versehentliches Eingeben nicht beabsichtigter Befehele auf der Eingabeeinheit bei einem Transport der Vorrichtung verhindert werden.

Wie erwähnt, kann die Eingabeeinheit gemäß einem Aspekt der Offenbarung einen berührungsempfindlichen Bildschirm aufweisen oder als solcher ausgebildet sein. Der Bildschirm kann als resistiver Touchscreen, kapazitiver Touchscreen, kraftbasierter Touchscreen (Piezo) oder als ultraschallbasierter Touchscreen ausgebildet sein.

Die vorliegende Offenbarung betrifft des Weiteren ein Verfahren zum Betreiben einer Vorrichtung zur Verabreichung von medizinischer Flüssigkeit.

Die zu betreibende Vorrichtung ist ausgestattet mit einem Hauptkörper, der einen Aufnahmebereich und einen Antrieb aufweist, wobei der Aufnahmebereich geeignet ist, einen flüssigkeitsbeinhaltenden Hohlkörper zumindest abschnittsweise aufzunehmen, und der Antrieb geeignet ist, auf einen zumindest abschnittsweise aufgenommenen Hohlkörper derart einzuwirken, dass eine in dem zumindest abschnittsweise aufgenommenen Hohlkörper beinhaltete Flüssigkeit gefördert wird. Eine Abdeckung ist mit dem Hauptkörper beweglich verbunden. Die Abdeckung deckt in einer geschlossenen Stellung den Aufnahmebereich nach außen hin ab, wobei an der Abdeckung eine manuell sperrbare Eingabeeinheit zur manuellen Eingabe von Befehlen an die Steuereinheit derart vorgesehen ist, dass in der geschlossenen Stellung der Abdeckung von außen zumindest abschnittsweise eine manuelle Eingabe auf der Eingabeeinheit möglich ist.

Das offenbarungsgemäße Verfahren zum Betreiben der genannten Vorrichtung weist die Schritte auf:
- Erkennen, ob die Abdeckung in der geschlossenen Stellung ist,
- Ermitteln, ob die Eingabeeinheit bei einem erkannten Öffnen der Abdeckung gesperrt ist, und
- Sperren der Eingabeeinheit, falls die Eingabeeinheit bei dem erkannten Öffnen der Abdeckung nicht gesperrt ist.

Die vorliegende Offenbarung betrifft des Weiteren ein Verfahren zum Betreiben einer Vorrichtung zur Verabreichung von medizinischer Flüssigkeit mit einer verriegelbaren Abdeckung.

Die zu betreibende Vorrichtung ist ausgestattet mit einem Hauptkörper, der einen Aufnahmebereich und einen Antrieb aufweist, wobei der Aufnahmebereich geeignet ist, einen flüssigkeitsbeinhaltenden Hohlkörper zumindest abschnittsweise aufzunehmen, und der Antrieb geeignet ist, auf einen zumindest abschnittsweise aufgenommenen Hohlkörper derart einzuwirken, dass eine in dem zumindest abschnittsweise aufgenommenen Hohlkörper beinhaltete Flüssigkeit gefördert wird, einer mit dem Hauptkörper beweglich verbundenen Abdeckung, die in einer geschlossenen Stellung den Aufnahmebereich nach außen hin abdeckt, wobei an der Abdeckung eine manuell sperrbare Eingabeeinheit zur manuellen Eingabe von Befehlen an die Steuereinheit derart vorgesehen ist, dass in der geschlossenen Stellung der Abdeckung von außen zumindest abschnittsweise eine manuelle Eingabe auf der Eingabeeinheit möglich ist, und einer Verriegelungsvorrichtung, die geeignet ist, die Abdeckung in der geschlossenen Stellung zu verriegeln.

Das offenbarungsgemäße Verfahren zum Betreiben der genannten Vorrichtung weist die Schritte auf:
Erkennen, ob die Abdeckung in der geschlossenen Stellung verriegelt ist,
Ermitteln, ob die Eingabeeinheit bei einem erkannten Entriegeln der geschlossenen Abdeckung gesperrt ist, und
Sperren der Eingabeeinheit, falls die Eingabeeinheit bei dem erkannten Entriegeln der Abdeckung nicht gesperrt ist.
Kurzbeschreibung der Zeichnungen

Die vorliegende Erfindung wird im Folgenden anhand bevorzugter Ausführungsbeispiele unter Bezugnahme auf die beigefügten Zeichnungen näher beschrieben. Es zeigen:
Fig. 1 eine perspektivische Ansicht einer Vorrichtung gemäß einer ersten Ausführungsform der vorliegenden Offenbarung in Form einer Spritzenpumpe mit einer geschlossenen Abdeckung;
Fig. 2 eine perspektivische Ansicht der in Fig. 1 gezeigten Vorrichtung mit der Abdeckung in einer geöffneten Stellung;
Fig. 3 eine perspektivische Ansicht einer Vorrichtung gemäß einer zweiten Ausführungsform der vorliegenden Offenbarung in Form einer Infusionspumpe mit einer geschlossenen Abdeckung;
Fig. 4 eine perspektivische Ansicht der in Fig. 3 gezeigten Vorrichtung mit der Abdeckung in einer geöffneten Stellung;
Fig. 5 eine Frontansicht auf die in Fig. 4 gezeigten Vorrichtung gemäß Pfeil V in Fig. 4;
Fig. 6 eine Detailschnittansicht einer Verriegelungsvorrichtung gemäß Pfeil VI in Fig. 4 in einem Zustand kurz vor einem Schließen und Verriegeln einer Abdeckung; und
Fig. 7 eine Detailschnittansicht der Verriegelungsvorrichtung in einem verriegelten Zustand.

### Detaillierte Beschreibung bevorzugter Ausführungsformen

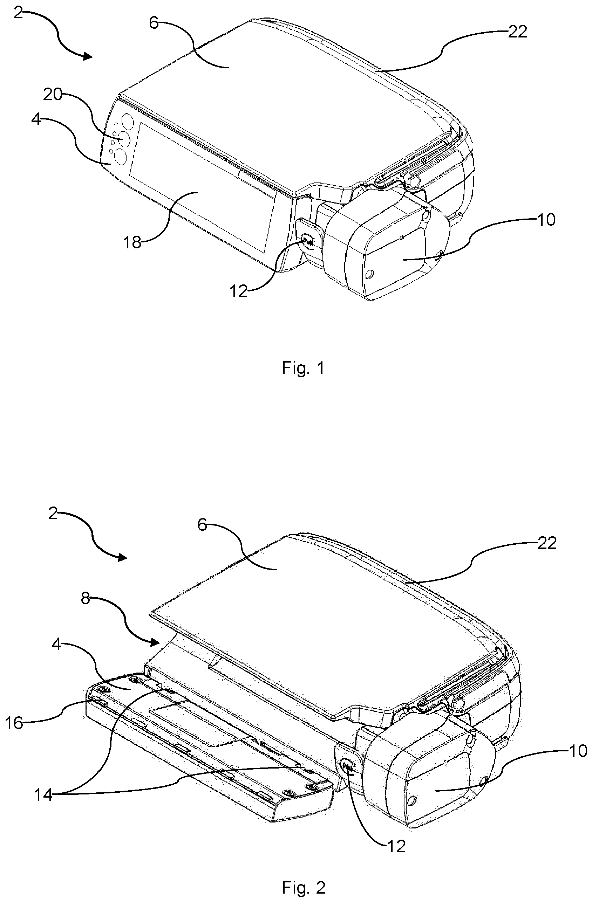

Fig. 1 und Fig. 2 zeigen perspektivische Ansichten einer Vorrichtung 2 gemäß einer ersten Ausführungsform der vorliegenden Offenbarung in Form einer Spritzenpumpe. In Fig. 1 ist die Vorrichtung 2 mit einer Abdeckung 4 in einer geschlossenen Stellung gezeigt. In Fig. 2 ist die Vorrichtung 2 mit der Abdeckung 4 in einer geöffneten Stellung gezeigt.

Die Vorrichtung 2 weist einen im Wesentlichen quaderförmigen Hauptkörper 6 auf. An einer langen Seitenfläche des Hauptkörpers 6, die in der Gebrauchslage der Vorrichtung 2 einem Benutzer zugewandt ist, weist die Vorrichtung 2 die Abdeckung 4 auf. Die der Abdeckung 4 zugewandte Seitenfläche des Hauptkörpers 6 weist einen Aufnahmebereich 8 auf. Der Aufnahmebereich 8 ist ausgebildet, eine (nicht gezeigte) Spritze derart aufzunehmen, dass ein Kolben der Spritze mittels eines an einer kurzen Seite des Aufnahmebereichs 8 angeordneten Schiebers 10 verschoben werden kann. Der Schieber 10 wird dazu mittels eines im Hauptkörper 6 integrierten Antriebs bewegt. Um eine Spritze in den Aufnahmebereich 8 auf einfache Weise zu montieren, ist an dem Schieber 10 ein federbelastetes Betätigungselement 12 vorgesehen, welches in einem betätigten Zustand den Schieber 10 derart vom Antrieb löst, dass der Schieber 10 manuell verschoben werden kann.

Die Abdeckung 4 ist über eine Scharniervorrichtung 14 schwenkbar mit einer in Gebrauchslage der Vorrichtung 2 unten angeordneten langen Kante des Hauptkörpers 6 verbunden.

An einer Innenseite der Abdeckung 4 sind an einer der Scharniervorrichtung 14 gegenüberliegenden langen Kante Federklemmen 16 vorgesehen, mittels derer die Abdeckung 4 in der geschlossenen Stellung mit dem Hauptkörper 6 verklemmt ist.

An einer der Innenseite gegenüberliegenden Außenseite der Abdeckung 4 ist ein sperrbarer berührungsempfindlicher Bildschirm 18 vorgesehen, der mit einer (nicht gezeigten) im Hauptkörper 6 vorgesehenen Steuereinheit verbunden ist. Der Bildschirm 18 hat den Umriss eines langgestreckten Rechtecks und erstreckt sich entlang der im Wesentlichen rechteckförmigen Abdeckung 4. An einer dem Schieber 10 gegenüberliegenden kurzen Kante des Bildschirms 18 sind mehrere mit der Steuereinheit verbundene Knöpfe 20 vorgesehen. Der Bildschirm 18 und die Knöpfe 20 bilden in der vorliegenden Ausführungsform die offenbarungsgemäße Eingabeeinheit.

In der in Fig. 1 gezeigten Stellung der Abdeckung 4 ist der Bildschirm 18 in der Regel entsperrt. Durch manuelles Eingeben einer Sperrungsbedienungssequenz auf dem Bildschirm 18 kann die Steuereinheit den Bildschirm 18 sperren. Die Sperrungsbedienungssequenz weist zumindest zwei Schritte auf, um eine Wahrscheinlichkeit für ein versehentliches Eingeben der Sperrungsbedienungssequenz zu verringern. Entsprechend kann durch manuelles Eingeben einer Entsperrungssequenz auf dem Bildschirm 18 der Bildschirm 18 entsperrt werden. Die Vorrichtung kann auch derart ausgebildet sein, dass zumindest einer der Knöpfe 20 bei der Sperrungsbedienungssequenz und/oder der Entsperrungsbedienungssequenz einbezogen ist, das heißt im Laufe der jeweiligen Sequenz gedrückt werden muss.

In der Abdeckung 4 ist zumindest ein (nicht gezeigter) Magnet vorgesehen. Im Hauptkörper 6 ist zumindest ein mit der Steuereinheit verbundener (nicht gezeigter) Hallsensor vorgesehen.

Wird die Abdeckung 4 aus der in Fig. 1 gezeigten geschlossenen Stellung geschwenkt, erkennt die Steuereinheit eine Änderung des Signals des Hallsensors und vermerkt, dass die Abdeckung 4 geöffnet ist. Die Vorrichtung kann auch derart ausgebildet sein, dass ein Taster/Drucksensor/Kontaktsensor in der Abdeckung 4 und/oder in dem Hauptkörper 6 eingebaut ist, und dass die Steuereinheit aufgrund der Kontaktierung des Tasters/Drucksensors/Kontaktsensors bei einem Schließen der Abdeckung 4 das Schließen und entsprechend das Öffnen der Abdeckung 4 erkennt.

Sobald die Abdeckung 4 von der Steuereinheit als geöffnet vermerkt ist, überprüft bzw. ermittelt die Steuereinheit, ob der Bildschirm 18 gesperrt ist. Wenn der Bildschirm 18 bei Erkennung der geöffneten Abdeckung 4 bereits gesperrt ist, belässt die Steuereinheit den Bildschirm 18 im gesperrten Zustand. Wenn der Bildschirm 18 bei Erkennung der geöffneten Abdeckung 4 nicht gesperrt ist, sperrt die Steuereinheit den Bildschirm 18.

Die Vorrichtung 2 weist einen schwenkbaren U-förmigen Tragbügel 22 auf. Die freien Enden des Tragbügels 22 sind an einer Oberseite des Hauptkörpers 6, jeweils mittig an den kurzen Kanten schwenkbar mit dem Hauptkörper 6 verbunden. In einer in Fig. 1 und Fig. 2 gezeigten eingeklappten Stellung erstreckt sich der Tragbügel 22 entlang der Kanten des Hauptkörpers 6. In einer (nicht gezeigten) ausgeklappten Stellung ist der Tragbügel 22 von dem Hauptkörper 6 abgespreizt und bietet einen Griff zum Tragen der Vorrichtung 2.

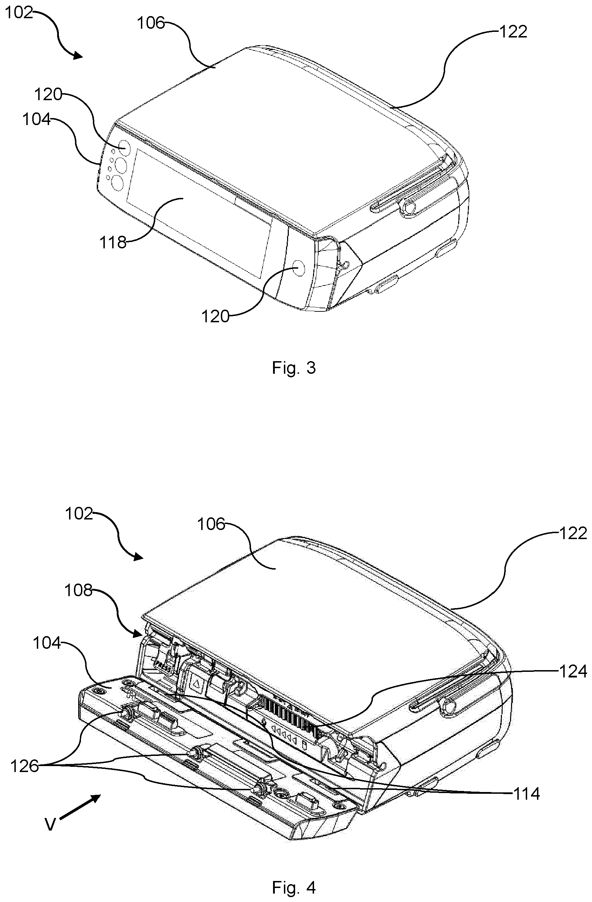

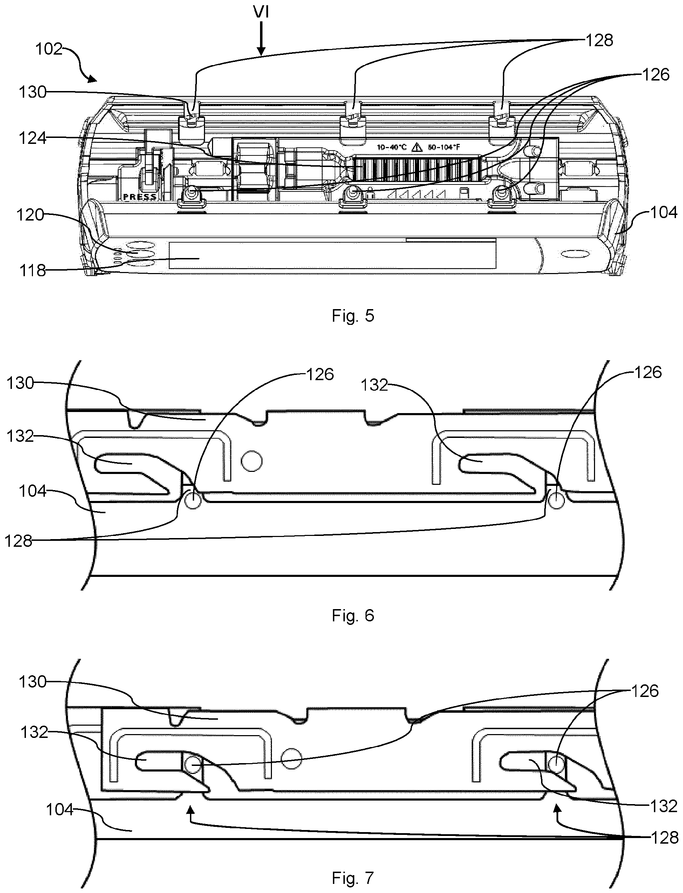

In Fig. 3 und Fig. 4 sind perspektivische Ansichten einer Vorrichtung 102 gemäß einer zweiten Ausführungsform der vorliegenden Offenbarung in Form einer Infusionspumpe gezeigt. In Fig. 3 ist die Vorrichtung 102 mit einer Abdeckung 104 in einer geschlossenen Stellung gezeigt. In Figur 4 ist die Vorrichtung 102 mit der Abdeckung 104 in einer geöffneten Stellung gezeigt. Fig. 5 zeigt eine Frontansicht der Vorrichtung 102 mit geöffneter Abdeckung 104. Fig. 6 und Fig. 7 zeigen Schnittansichten eines Teils einer Verriegelungsvorrichtung für die Abdeckung 104 der Vorrichtung 102 in einem entriegelten Zustand (Fig. 6) und einem verriegelten Zustand (Fig. 7).

Die Vorrichtung 102 weist einen im Wesentlichen quaderförmigen Hauptkörper 106 auf. An einer langen Seitenfläche des Hauptkörpers 106, die in der Gebrauchslage der Vorrichtung 102 einem Benutzer zugewandt ist, weist die Vorrichtung 102 die Abdeckung 104 auf. Die der Abdeckung 104 zugewandte Seitenfläche des Hauptkörpers 106 weist einen Aufnahmebereich 108 auf. Der Aufnahmebereich 108 ist ausgebildet, einen (nicht gezeigten) Schlauch aufzunehmen, und weist eine Peristaltik 124 auf, mittels derer nebeneinander angeordnete Abschnitte des Schlauchs nacheinander abgedrückt werden können, um so eine von dem Schlauch beinhaltete Flüssigkeit fördern zu können.

Die Abdeckung 104 ist über eine Scharniervorrichtung 114 schwenkbar mit einer in Gebrauchslage der Vorrichtung 102 vorne unten angeordneten langen Kante des Hauptkörpers 106 verbunden.

An einer Innenseite der Abdeckung 104 sind an einer der Scharniervorrichtung 114 gegenüberliegenden langen Kante Sperrstifte 126 vorgesehen, die in der geschlossenen Stellung der Abdeckung 104 in entsprechende Aufnahmen 128 an einer in Gebrauchslage der Vorrichtung 102 vorderen oberen Kante des Hauptkörpers 106 eintauchen und mittels eines (in den Figuren 5, 6 und 7 gezeigten) Riegels 130 in den Aufnahmen 128 fixiert werden können, um so die die Abdeckung 104 in der geschlossenen Stellung zu verriegeln. Wie in Fig. 6 und Fig. 7 gezeigt, weist der Riegel 130 je Sperrstift 126 eine hakenförmige Hinterschneidung 132 auf. In der geschlossenen Stellung der Abdeckung 104 sind die Sperrstifte 126 in den entsprechenden Aufnahmen 128 aufgenommen. Wird nun der Riegel 130 von der entriegelten Stellung in die verriegelte Stellung gebracht, werden die Sperrstifte 126 zusätzlich von den hakenförmigen Hinterschneidungen 132 des Riegels 130 aufgenommen, so dass die Aufnahmen 128 versperrt sind und die Sperrstifte 126 in den Aufnahmen 128 gefangen sind.

An einer der Innenseite gegenüberliegenden Außenseite der Abdeckung 104 ist ein sperrbarer berührungsempfindlicher Bildschirm 118 vorgesehen, der mit einer (nicht gezeigten) im Hauptkörper 106 vorgesehenen Steuereinheit verbunden ist. Der Bildschirm 118 hat den Umriss eines langgestreckten Rechtecks und erstreckt sich entlang der im Wesentlichen rechteckförmigen Abdeckung 104. An den kurzen Kanten des Bildschirms 118 sind mehrere mit der Steuereinheit verbundene Knöpfe 120 vorgesehen. Der Bildschirm 118 und die Knöpfe 120 bilden in der vorliegenden Ausführungsform die offenbarungsgemäße Eingabeeinheit.

In der in Fig. 3 gezeigten Stellung der Abdeckung 104 ist der Bildschirm 118 in der Regel entsperrt. Durch manuelles Eingeben einer Sperrungsbedienungssequenz auf dem Bildschirm 118 kann die Steuereinheit den Bildschirm 118 sperren. Die Sperrungsbedienungssequenz weist zumindest zwei Schritte auf, um eine Wahrscheinlichkeit für ein versehentliches Eingeben der Sperrungsbedienungssequenz zu verringern. Entsprechend kann durch manuelles Eingeben einer Entsperrungssequenz auf dem Bildschirm 118 der Bildschirm 118 entsperrt werden. Die Vorrichtung kann auch derart ausgebildet sein, dass zumindest einer der Knöpfe 120 bei der Sperrungsbedienungssequenz und/oder der Entsperrungsbedienungssequenz einbezogen ist, das heißt im Laufe der jeweiligen Sequenz gedrückt werden muss.

Der zum Fixieren der Sperrstifte 126 vorgesehenen Riegel 130 ist mit einem (nicht gezeigten) Potentiometer gekoppelt, mittels dessen die Steuereinheit die Stellung des Riegels 130 relativ zur Abdeckung 104 ermitteln kann. Die Ermittlung der Stellung der Abdeckung 104 gegenüber dem Hauptkörper 106 kann entsprechend der Vorrichtung 2 umgesetzt sein, das heißt mithilfe eines Hallsensors oder mithilfe eines Tasters/Drucksensors/Kontaktsensors. Die Vorrichtung 102 kann alternativ auch derart ausgebildet sein, dass die Stellung des Riegels 130 entsprechend der Stellung der Abdeckung 104 mithilfe eines Hallsensors, eines Tasters oder einer Lichtschranke ermittelt wird. Das nicht gezeigte Potentiometer und der nicht gezeigte Sensor zur Ermittlung der Stellung der Abdeckung bilden zusammen die offenbarungsgemäße Sensoreinheit.

Um die Abdeckung 104 aus der in Fig. 3 gezeigten geschlossenen Stellung in die in Fig. 4 gezeigte Stellung zu schwenken, muss ein unter den Knöpfen 120 dafür vorbestimmter Knopf gedrückt werden, der über die Steuereinheit ein Verschieben des zum Fixieren der Sperrstifte 126 vorgesehenen Riegels 130 veranlasst. Die Steuereinheit überwacht die Stellung der Abdeckung 104 relativ zum Hauptkörper 106 und erkennt eine Änderung des Signals des Potentiometers und vermerkt, dass die Abdeckung 104 "verriegelt und geschlossen", "entriegelt und geschlossen" oder "entriegelt und geöffnet" ist.

Sobald die Abdeckung 104 von der Steuereinheit als entriegelt vermerkt ist, das heißt, sobald der Zustand "verriegelt und geschlossen" nicht mehr vorliegt, überprüft die Steuereinheit, ob der Bildschirm 118 gesperrt ist. Wenn der Bildschirm 118 bei Erkennung der entriegelten Abdeckung 104 bereits gesperrt ist, belässt die Steuereinheit den Bildschirm 118 im gesperrten Zustand. Wenn der Bildschirm 118 bei Erkennung der entriegelten Abdeckung 104 nicht gesperrt ist, sperrt die Steuereinheit den Bildschirm 118.

Die Vorrichtung 102 weist einen schwenkbaren U-förmigen Tragbügel 122 auf. Die freien Enden des Tragbügels 122 sind an einer Oberseite des Hauptkörpers 106, jeweils mittig an den kurzen Kanten schwenkbar mit dem Hauptkörper 106 verbunden. In einer in Fig. 3 und Fig. 4 gezeigten eingeklappten Stellung erstreckt sich der Tragbügel 122 entlang der Kanten des Hauptkörpers 106. In einer (nicht gezeigten) ausgeklappten Stellung ist der Tragbügel 122 von dem Hauptkörper 106 abgespreizt und bietet einen Griff zum Tragen der Vorrichtung 102.

Die in den Figuren 1 bis 7 gezeigten und oben beschriebenen Ausführungsformen der offenbarungsgemäßen Vorrichtung stellen lediglich zwei mögliche Umsetzungen der beanspruchten Erfindung dar.

Gemäß den beschriebenen Ausführungsformen werden die Sperrungsbedienungssequenz und die Entsperrungsbedienungssequenz der Steuereinheit durch Eingabe auf dem Bildschirm 18 bzw. 118 mittgeteilt. Alternativ ist es möglich, die Sperrungsbedienungssequenz und die Entsperrungsbedienungssequenz über die Knöpfe 20 bzw. 120 einzugeben. Alternativ ist es auch möglich die Sperrungsbedienungssequenz und die Entsperrungsbedienungssequenz jeweils teils durch Betätigung zumindest eines der Knöpfe 20 bzw. 120 und teils durch Betätigung zumindest einer auf dem Bildschirm 18 bzw. 118 angezeigten Betätigungsfläche der Steuereinheit mitzuteilen.

Die Vorrichtung 2 bzw. 102 kann mit einem Sensor ausgestattet sein, der geeignet ist, ein Ausklappen des Tragbügels 22 bzw. 122 zu erkennen. Die Steuereinheit kann ausgebildet sein, bei ausgeklappten Tragbügel 22 bzw. 122 den Bildschirm 18 bzw. 118 zu sperren.

### Bezugszeichenliste

- 2; 102: Vorrichtung
- 4; 104: Abdeckung
- 6; 106: Hauptkörper
- 8; 108: Aufnahmebereich
- 10: Schieber
- 12: Betätigungselement
- 14; 114: Scharniervorrichtung
- 16: Federklemme
- 18; 118: Bildschirm
- 20; 120: Knopf
- 22; 122: Tragbügel
- 124: Peristaltik
- 126: Sperrstift
- 128: Aufnahme
- 130: Riegel
- 132: hakenförmige Hinterschneidung

## Patentansprüche

1. Vorrichtung (2) zur Verabreichung von medizinischer Flüssigkeit mit
einem Hauptkörper (6), der einen Aufnahmebereich (8) und einen Antrieb aufweist, wobei der Aufnahmebereich (8) geeignet ist, einen flüssigkeitsbeinhaltenden Hohlkörper zumindest abschnittsweise aufzunehmen, und der Antrieb geeignet ist, auf einen zumindest abschnittsweise aufgenommenen Hohlkörper derart einzuwirken, dass eine in dem zumindest abschnittsweise aufgenommenen Hohlkörper beinhaltete Flüssigkeit gefördert wird,
einer Steuereinheit zur Steuerung des Antriebs,
einer mit dem Hauptkörper (6) beweglich verbundenen Abdeckung (4), die in einer geschlossenen Stellung den Aufnahmebereich (8) nach außen hin abdeckt,
einer Eingabeeinheit (18) zur manuellen Eingabe von Befehlen an die Steuereinheit, und
einem Sensor, der geeignet ist, zu erkennen, ob die Abdeckung (4) in der geschlossenen Stellung ist,
**dadurch gekennzeichnet, dass**
die Eingabeeinheit (18) derart an der Abdeckung (4) vorgesehen ist, dass in der geschlossenen Stellung der Abdeckung (4) von außen zumindest abschnittsweise eine manuelle Eingabe auf der Eingabeeinheit (18) möglich ist,
die Eingabeeinheit (18) manuell sperrbar ist und
die Steuereinheit ausgebildet ist, bei einem von dem Sensor erkannten Öffnen der Abdeckung (4) zu ermitteln, ob die Eingabeeinheit (18) gesperrt ist, und, falls die Eingabeeinheit (18) bei dem erkannten Öffnen der Abdeckung (4) nicht gesperrt ist, die Eingabeeinheit (18) zu sperren.

2. Vorrichtung (2) nach Anspruch 1, **dadurch gekennzeichnet, dass** die Steuereinheit ausgebildet ist, nach einem Öffnen der Abdeckung (4) und einem dadurch verursachten Sperren der Eingabeeinheit bei einem von dem Sensor erkannten Schließen der Abdeckung (4) die Eingabeeinheit zu entsperren.

3. Vorrichtung (2) nach Anspruch 1 oder 2, **dadurch gekennzeichnet, dass** die Steuereinheit ausgebildet ist, nach einer nicht durch ein Öffnen der Abdeckung (4) verursachten Sperrung der Eingabeeinheit und einem Öffnen der Abdeckung (4) bei einem von dem Sensor erkannten Schließen der Abdeckung (4) die Sperrung der Eingabeeinheit (18) aufrechtzuerhalten.

4. Vorrichtung (2) nach einem der Ansprüche 1 bis 3, **dadurch gekennzeichnet, dass** die Steuereinheit ausgebildet ist, eine vor einem Öffnen der Abdeckung (4) begonnene und bei dem Öffnen der Abdeckung (4) andauernde manuelle Eingabe aufzuheben.

5. Vorrichtung (2) nach einem der Ansprüche 1 bis 4, **dadurch gekennzeichnet, dass**
die Vorrichtung (2) einen Sensor aufweist, der geeignet ist, zu erkennen, ob ein Hohlkörper in dem Aufnahmebereich (8) aufgenommen ist, und
die Steuereinheit ausgebildet ist, ein von der Stellung der Abdeckung (4) abhängiges Entsperren und/oder Sperren der Eingabeeinheit nur bei Erkennung eines in dem Aufnahmebereich (8) aufgenommenen Hohlkörpers durchzuführen.

6. Vorrichtung (102) zur Verabreichung von medizinischer Flüssigkeit mit
einem Hauptkörper (106), der einen Aufnahmebereich (108) und einen Antrieb aufweist, wobei der Aufnahmebereich (108) geeignet ist, einen flüssigkeitsbeinhaltenden Hohlkörper zumindest abschnittsweise aufzunehmen, und der Antrieb geeignet ist, auf einen zumindest abschnittsweise aufgenommenen Hohlkörper derart einzuwirken, dass eine in dem zumindest abschnittsweise aufgenommenen Hohlkörper beinhaltete Flüssigkeit gefördert wird,
einer Steuereinheit zur Steuerung des Antriebs,
einer mit dem Hauptkörper beweglich verbundenen Abdeckung (104), die in einer geschlossenen Stellung den Aufnahmebereich (108) nach außen hin abdeckt,
einer Eingabeeinheit zur manuellen Eingabe von Befehlen an die Steuereinheit,
einer Verriegelungsvorrichtung, die geeignet ist, die Abdeckung (104) in der geschlossenen Stellung zu verriegeln, und
einer Sensoreinheit, die geeignet ist, zu erkennen, ob die Abdeckung (104) in der geschlossenen Stellung verriegelt ist,
**dadurch gekennzeichnet, dass**
die Eingabeeinheit derart an der Abdeckung (104) vorgesehen ist, dass in der geschlossenen Stellung der Abdeckung (104) von außen zumindest abschnittsweise eine manuelle Eingabe auf der Eingabeeinheit möglich ist,
die Eingabeeinheit manuell sperrbar ist und
die Steuereinheit ausgebildet ist, bei einem von der Sensoreinheit erkannten Entriegeln der geschlossenen Abdeckung (104) zu ermitteln, ob die Eingabeeinheit gesperrt ist, und, falls die Eingabeeinheit bei dem erkannten Entriegeln der Abdeckung (104) nicht gesperrt ist, die Eingabeeinheit zu sperren.

7. Vorrichtung (102) nach Anspruch 6, **dadurch gekennzeichnet, dass** die Steuereinheit ausgebildet ist, nach einem Entriegeln und Öffnen der Abdeckung (104) und einem durch das Entriegeln verursachten Sperren der Eingabeeinheit bei einem von der Sensoreinheit erkannten Schließen und Verriegeln der Abdeckung (104) die Eingabeeinheit zu entsperren.

8. Vorrichtung (102) nach Anspruch 7, **dadurch gekennzeichnet, dass** die Steuereinheit ausgebildet ist, nach einer nicht durch ein Entriegeln der Abdeckung (104) verursachten Sperrung der Eingabeeinheit und einem Entriegeln der Abdeckung (104) bei einem von dem Sensor erkannten Verriegeln der Abdeckung (104) die Sperrung der Eingabeeinheit aufrechtzuerhalten.

9. Vorrichtung (102) nach einem der Ansprüche 6 bis 8, **dadurch gekennzeichnet, dass** die Steuereinheit ausgebildet ist, eine vor einem Entriegeln der Abdeckung (104) begonnene und bei dem Entriegeln der Abdeckung (104) andauernde manuelle Eingabe aufzuheben.

10. Vorrichtung (102) nach einem der Ansprüche 6 bis 9, **dadurch gekennzeichnet, dass**
die Vorrichtung (102) einen Sensor aufweist, der geeignet ist, zu erkennen, ob ein Hohlkörper in dem Aufnahmebereich (108) aufgenommen ist, und
die Steuereinheit ausgebildet ist, ein von der Verriegelung der geschlossenen Abdeckung (104) abhängiges Entsperren und/oder Sperren der Eingabeeinheit nur bei Erkennung eines in dem Aufnahmebereich (108) aufgenommenen Hohlkörpers durchzuführen.

11. Vorrichtung (2; 102) nach einem der Ansprüche 1 bis 10, **dadurch gekennzeichnet, dass** die Steuereinheit ausgebildet ist, die Eingabeeinheit nach einer Entsperrung und eines darauffolgenden Ablaufs eines Zeitintervalls, in dem keine manuelle Eingabe auf der Eingabeeinheit erfolgt, zu sperren.

12. Vorrichtung (2; 102) nach einem der Ansprüche 1 bis 11, **dadurch gekennzeichnet, dass**
die Vorrichtung (2) einen Lagesensor aufweist, der geeignet ist, eine Lageänderung des Hauptkörpers (6) zu erkennen, und
die Steuereinheit ausgebildet ist, bei einem Überschreiten der erkannten Lageänderung über einen vorbestimmten Schwellenwert zu ermitteln, ob die Eingabeeinheit gesperrt ist, und, falls die Eingabeeinheit nicht gesperrt ist, die Eingabeeinheit zu sperren.

13. Vorrichtung (2; 102) nach einem der Ansprüche 1 bis 12, **dadurch gekennzeichnet, dass** die Eingabeeinheit einen sperrbaren berührungsempfindlichen Bildschirm (18; 118) aufweist.

14. Verfahren zum Betreiben einer Vorrichtung (2) zur Verabreichung von medizinischer Flüssigkeit mit einem Hauptkörper (6), der einen Aufnahmebereich (8) und einen Antrieb aufweist, wobei der Aufnahmebereich (8) geeignet ist, einen flüssigkeitsbeinhaltenden Hohlkörper zumindest abschnittsweise aufzunehmen, und der Antrieb geeignet ist, auf einen zumindest abschnittsweise aufgenommenen Hohlkörper derart einzuwirken, dass eine in dem zumindest abschnittsweise aufgenommenen Hohlkörper beinhaltete Flüssigkeit gefördert wird, und einer mit dem Hauptkörper (6) beweglich verbundenen Abdeckung (4), die in einer geschlossenen Stellung den Aufnahmebereich (8) nach außen hin abdeckt, wobei an der Abdeckung (4) eine manuell sperrbare Eingabeeinheit (18) zur manuellen Eingabe von Befehlen an die Steuereinheit derart vorgesehen ist, dass in der geschlossenen Stellung der Abdeckung (4) von außen zumindest abschnittsweise eine manuelle Eingabe auf der Eingabeeinheit möglich ist, mit dem Schritt
Erkennen, ob die Abdeckung (4) in der geschlossenen Stellung ist,
**gekennzeichnet, durch** die Schritte
Ermitteln, ob die Eingabeeinheit bei einem erkannten Öffnen der Abdeckung (4) gesperrt ist, und
Sperren der Eingabeeinheit, falls die Eingabeeinheit bei dem erkannten Öffnen der Abdeckung (4) nicht gesperrt ist.

15. Verfahren zum Betreiben einer Vorrichtung (102) zur Verabreichung von medizinischer Flüssigkeit mit einem Hauptkörper (106), der einen Aufnahmebereich (108) und einen Antrieb aufweist, wobei der Aufnahmebereich (108) geeignet ist, einen flüssigkeitsbeinhaltenden Hohlkörper zumindest abschnittsweise aufzunehmen, und der Antrieb geeignet ist, auf einen zumindest abschnittsweise aufgenommenen Hohlkörper derart einzuwirken, dass eine in dem zumindest abschnittsweise aufgenommenen Hohlkörper beinhaltete Flüssigkeit gefördert wird, einer mit dem Hauptkörper beweglich verbundenen Abdeckung (104), die in einer geschlossenen Stellung den Aufnahmebereich (108) nach außen hin abdeckt, wobei an der Abdeckung (104) eine manuell sperrbare Eingabeeinheit zur manuellen Eingabe von Befehlen an die Steuereinheit derart vorgesehen ist, dass in der geschlossenen Stellung der Abdeckung (104) von außen zumindest abschnittsweise eine manuelle Eingabe auf der Eingabeeinheit möglich ist, und einer Verriegelungsvorrichtung, die geeignet ist, die Abdeckung (104) in der geschlossenen Stellung zu verriegeln, mit dem Schritt
Erkennen, ob die Abdeckung (104) in der geschlossenen Stellung verriegelt ist,
**gekennzeichnet, durch** die Schritte
Ermitteln, ob die Eingabeeinheit bei einem erkannten Entriegeln der geschlossenen Abdeckung (104) gesperrt ist, und
Sperren der Eingabeeinheit, falls die Eingabeeinheit bei dem erkannten Entriegeln der Abdeckung (4) nicht gesperrt ist.

## Claims

1. A device (2) for administering a medical liquid, comprising
a main part (6), which has a receiving region (8) and a drive, the receiving region (8) being suitable for at least partly receiving a liquid-containing hollow body, and the drive being suitable for acting on an at least partly received hollow body such that a liquid contained in the at least partly received hollow body is conveyed,
a control unit for controlling the drive,
a cover (4), which is movably connected to the main part (6) and covers the exterior of the receiving region (8) in a closed position,
an input unit (18) for manually inputting commands on the control unit, and
a sensor, which is suitable for detecting whether the cover (4) is in the closed position,
**characterized in that**
the input unit (18) is provided on the cover (4) in such a way that, in the closed position of the cover (4), manual input on the input unit (18) is possible from the outside, at least in sections,
the input unit (18) is manually blockable, and
the control unit is designed to determine, when the sensor detects that the cover (4) is opened, whether the input unit (18) is blocked, and, if the input unit (18) is not blocked when the cover (4) is detected to be opened, to block the input unit (18).

2. The device (2) according to claim 1, **characterized in that** the control unit is designed to unblock the input unit after opening of the cover (4) and a thereby caused blocking of the input unit, when the sensor detects that the cover (4) is closed.

3. The device (2) according to claim 1 or 2, **characterized in that** the control unit is designed to maintain the blocking of the input unit (18), after blocking of the input unit which is not caused by opening of the cover (4) and opening of the cover (4) when the sensor detects that the cover (4) is closed.

4. The device (2) according to any one of the claims 1 to 3, **characterized in that** the control unit is designed to cancel a manual input which was started prior to the opening of the cover (4) and continues when the cover (4) is opened.

5. The device (2) according to any one of the claims 1 to 4, **characterized in that**
the device (2) includes a sensor which is suitable for detecting whether a hollow body is received in the receiving region (8), and
the control unit is designed to carry out unblocking and/or blocking of the input unit depending on the position of the cover (4) only when a hollow body received in the receiving region (8) is detected.

6. A device (102) for administering a medical liquid comprising:
a main part (106), which has a receiving region (108) and a drive, the receiving region (108) being suitable for receiving at least partly a liquid-containing hollow body, and the drive being suitable for acting on an at least partly received hollow body such that a liquid contained in the at least partly received hollow body is conveyed,
a control unit for controlling the drive,
a cover (104), which is movably connected to the main part and covers the exterior of the receiving region (108) in a closed position,
an input unit for manually inputting commands on the control unit, and
a locking device, which is suitable for locking the cover (104) in the closed position, and
a sensor unit, which is suitable for detecting whether the cover (104) is locked in the closed position,
**characterized in that**
the input unit is provided on the cover (104) in such a way that, in the closed position of the cover (104), manual input on the input unit is possible from the outside, at least in sections,
the input unit is manually lockable, and
the control unit is designed to determine, when the sensor unit detects that the closed cover (104) is unlocked, whether the input unit is blocked, and, if the input unit is not blocked when the unlocking of the cover (104) is detected, to block the input unit.

7. The device (102) according to claim 6, **characterized in that** the control unit is designed to unblock the input unit after unlocking and opening of the cover (104) and blocking of the input unit which is caused by unlocking, when the sensor unit detects that the cover (104) is closed and locked.

8. The device (102) according to claim 7, **characterized in that** the control unit is designed to maintain the blocking of the input unit after blocking of the input unit which is not caused by unlocking of the cover (104) and unlocking of the cover (104) when the sensor detects that the cover (104) is locked.

9. The device (102) according to any one of the claims 6 to 8, **characterized in that** the control unit is designed to cancel a manual input which was started before unlocking of the cover (104) and continues when the cover (104) is unlocked.

10. The device (102) according to any one of the claims 6 to 9, **characterized in that**
the device (102) comprises a sensor which is suitable for detecting whether a hollow body is received in the receiving region (108), and
the control unit is designed to carry out unblocking and/or blocking of the input unit depending on the locking of the closed cover (104) only when a hollow body received in the receiving region (108) is detected.

11. The device (2; 102) according to any one of the claims 1 to 10, **characterized in that** the control unit is designed to block the input unit after unblocking and a subsequent expiry of a time interval in which no manual input is carried out on the input unit.

12. The device (2; 102) according to any one of the claims 1 to 11, **characterized in that**
the device (2) comprises a position sensor which is suitable for detecting a change of position of the main part (6), and
the control unit is designed to determine, when the detected change of position exceeds a predetermined threshold, whether the input unit is blocked, and if the input unit is not blocked, to block the input unit.

13. The device (2; 102) according to any one of the claims 1 to 12, **characterized in that** the input unit includes a blockable touch-sensitive screen (18; 118).

14. A method for operating a device (2) for administering a medical liquid comprising a main part (6), which has a receiving region (8) and a drive, the receiving region (8) being suitable for at least partly receiving a liquid-containing hollow body, the drive being suitable for acting on an at least partly received hollow body such that a liquid contained in the at least partly received hollow body is conveyed, and a cover (4), which is movably connected to the main part (6) and covers the exterior of the receiving region (8) in a closed position, wherein a manually blockable input unit (18) for manually inputting commands on the control unit is provided on the cover (4) such that in the closed position of the cover (4) it is at least partly possible to carry out a manual input on the input unit from the outside, the method comprising:
detecting whether or not the cover (4) is in the closed position,
**characterized by** further comprising:
determining whether or not the input unit is blocked when it is detected that the cover (4) is opened, and
blocking the input unit, if the input unit is not blocked when the opening of the cover (4) is detected.

15. A method for operating a device (102) for administering a medical liquid comprising a main part (106), which has a receiving region (108) and a drive, the receiving region (108) being suitable for at least partly receiving a liquid-containing hollow body, and the drive being suitable for acting on an at least partly received hollow body such that a liquid contained in the at least partly received hollow body is conveyed, and a cover (104), which is movably connected to the main part and which covers the exterior of the receiving region (108) in a closed position, wherein a manually blockable input unit for manually inputting commands on the control unit is provided on the cover (104) such that in the closed position of the cover (104) it is at least partly possible to carry out a manual input on the input unit from the outside, and a locking device which is suitable for locking the cover (104) in the closed position, the method comprising:
detecting whether the cover (104) is locked in the closed position,
**characterized by** further comprising
determining whether the input unit is blocked when it is detected that the closed cover (104) is unlocked, and
blocking the input unit, if the input unit is not blocked when the unlocking of the cover (104) is detected.

## Revendications

1. Dispositif (2) pour l'administration de liquide médical avec
un corps principal (6) qui présente une zone de réception (8) et un entraînement, dans lequel la zone de réception (8) est appropriée afin de recevoir au moins par sections un corps creux contenant du liquide, et l'entraînement est approprié afin d'agir sur un corps creux reçu au moins par sections de telle manière qu'un liquide contenu dans le corps creux reçu au moins par sections soit transporté,
un dispositif de commande pour la commande de l'entraînement,
un couvercle (4) relié de manière mobile au corps principal (6), couvercle qui recouvre vers l'extérieur la zone de réception (8) dans une position fermée,
une unité de saisie (18) pour la saisie manuelle d'ordres au niveau du dispositif de commande, et
un capteur qui est approprié afin de reconnaître si le couvercle (4) est dans la position fermée,
**caractérisé en ce que**
l'unité de saisie (18) est prévue au niveau du couvercle (4) de telle manière que dans la position fermée du couvercle (4), une saisie manuelle sur l'unité de saisie (18) soit possible de l'extérieur au moins par sections,
l'unité de saisie (18) peut être bloquée manuellement et
le dispositif de commande est réalisé afin de déterminer, en cas d'ouverture du couvercle (4) détectée par le capteur, si l'unité de saisie (18) est bloquée, et, si l'unité de saisie (18) n'est pas bloquée en cas d'ouverture détectée du couvercle (4), de bloquer l'unité de saisie (18).

2. Dispositif (2) selon la revendication 1, **caractérisé en ce que** le dispositif de commande est réalisé afin de débloquer l'unité de saisie après une ouverture du couvercle (4) et un blocage de l'unité de saisie causé par celle-ci en cas de fermeture détectée par le capteur du couvercle (4).

3. Dispositif (2) selon la revendication 1 ou 2, **caractérisé en ce que** le dispositif de commande est réalisé afin de maintenir le blocage de l'unité de saisie (18) après un blocage non causé par une ouverture du couvercle (4) de l'unité de saisie et une ouverture du couvercle (4) en cas de fermeture détectée par le capteur du couvercle (4).

4. Dispositif (2) selon l'une quelconque des revendications 1 à 3, **caractérisé en ce que** le dispositif de commande est réalisé afin de supprimer une saisie manuelle commencée avant une ouverture du couvercle (4) et qui se poursuit lors de l'ouverture du couvercle (4).

5. Dispositif (2) selon l'une quelconque des revendications 1 à 4, **caractérisé en ce que**
le dispositif (2) présente un capteur qui est approprié afin de détecter si un corps creux est reçu dans la zone de réception (8), et
le dispositif de commande est réalisé afin de mettre en oeuvre un déblocage et/ou blocage dépendant de la position du couvercle (4) de l'unité de saisie uniquement en cas de détection d'un corps creux reçu dans la zone de réception (8).

6. Dispositif (102) pour l'administration de liquide médical avec
un corps principal (106) qui présente une zone de réception (108) et
un entraînement, dans lequel la zone de réception (108) est appropriée afin de recevoir au moins par sections un corps creux contenant du liquide, et l'entraînement est approprié afin d'agir sur un corps creux reçu au moins par sections de telle manière qu'un liquide contenu dans le corps creux reçu au moins par sections soit transporté,
un dispositif de commande pour la commande de l'entraînement,
un couvercle (104) relié de manière mobile au corps principal, couvercle qui recouvre vers l'extérieur la zone de réception (108) dans une position fermée,
une unité de saisie pour la saisie manuelle d'ordres au niveau du dispositif de commande,
un dispositif de verrouillage qui est approprié afin de verrouiller le couvercle (104) dans la position fermée, et
une unité de capteur qui est appropriée afin de détecter si le couvercle (104) est verrouillé dans la position fermée,
**caractérisé en ce que**
l'unité de saisie est prévue au niveau du couvercle (104) de telle manière que dans la position fermée du couvercle (104), une saisie manuelle sur l'unité de saisie soit possible de l'extérieur au moins par sections,
l'unité de saisie peut être bloquée manuellement et
le dispositif de commande est réalisé afin de déterminer, en cas de déverrouillage détecté par l'unité de capteur du couvercle (104) si l'unité de saisie est bloquée, et, si l'unité de saisie n'est pas bloquée en cas de déverrouillage détecté du couvercle (104), de bloquer l'unité de saisie.

7. Dispositif (102) selon la revendication 6, **caractérisé en ce que** le dispositif de commande est réalisé afin de débloquer l'unité de saisie après un déverrouillage et une ouverture du couvercle (104) et un blocage causé par le déverrouillage de l'unité de saisie en cas de fermeture détectée par l'unité de capteur et de verrouillage du couvercle (104).

8. Dispositif (102) selon la revendication 7, **caractérisé en ce que** le dispositif de commande est réalisé afin de maintenir le blocage de l'unité de saisie après un blocage non causé par un déverrouillage du couvercle (104) de l'unité de saisie et un déverrouillage du couvercle (104) en cas de verrouillage détecté par le capteur du couvercle (104).

9. Dispositif (102) selon l'une quelconque des revendications 6 à 8, **caractérisé en ce que** le dispositif de commande est réalisé afin de supprimer une saisie manuelle commencée avant un déverrouillage du couvercle (104) et qui se poursuit lors du déverrouillage du couvercle (104).

10. Dispositif (102) selon l'une quelconque des revendications 6 à 9, **caractérisé en ce que**
le dispositif (102) présente un capteur qui est approprié afin de détecter si un corps creux est reçu dans la zone de réception (108), et
le dispositif de commande est réalisé afin de mettre en oeuvre un déblocage et/ou blocage dépendant du verrouillage du couvercle (104) fermé de l'unité de saisie uniquement en cas de détection d'un corps creux reçu dans la zone de réception (108).

11. Dispositif (2 ; 102) selon l'une quelconque des revendications 1 à 10, **caractérisé en ce que** le dispositif de commande est réalisé afin de bloquer l'unité de saisie après un déverrouillage et un déroulement successif d'un intervalle de temps au cours duquel aucune saisie manuelle n'est effectuée sur l'unité de saisie.

12. Dispositif (2 ; 102) selon l'une quelconque des revendications 1 à 11, **caractérisé en ce que**
le dispositif (2) présente un capteur de position qui est approprié afin de détecter une modification de position du corps principal (6), et
le dispositif de commande est réalisé afin de déterminer, en cas de dépassement de la modification de position détectée au-dessus d'une valeur seuil prédéterminée si l'unité de saisie est bloquée, et, si l'unité de saisie n'est pas bloquée, de bloquer l'unité de saisie.

13. Dispositif (2 ; 102) selon l'une quelconque des revendications 1 à 12, **caractérisé en ce que** l'unité de saisie présente un écran (18 ; 118) tactile blocable.

14. Procédé de fonctionnement d'un dispositif (2) pour l'administration de liquide médical avec un corps principal (6) qui présente une zone de réception (8) et un entraînement, dans lequel la zone de réception (8) est appropriée afin de recevoir au moins par sections un corps creux contenant du liquide, et l'entraînement est approprié afin d'agir sur un corps creux reçu au moins par sections de telle manière qu'un liquide contenu dans le corps creux reçu au moins par sections soit transporté, et un couvercle (4) relié de manière mobile au corps principal (6), couvercle qui recouvre vers l'extérieur la zone de réception (8) dans une position fermée, dans lequel une unité de saisie (18) blocable manuellement est prévue au niveau du couvercle (4) pour la saisie manuelle d'ordres au niveau du dispositif de commande de telle manière que dans la position fermée du couvercle (4), une saisie manuelle sur l'unité de saisie soit possible de l'extérieur au moins par sections, avec l'étape consistant à
détecter si le couvercle (4) est dans la position fermée,
**caractérisé par** les étapes consistant à :
déterminer si l'unité de saisie est bloquée en cas d'ouverture détectée du couvercle (4), et
bloquer l'unité de saisie si l'unité de saisie n'est pas bloquée en cas d'ouverture détectée du couvercle (4).

15. Procédé de fonctionnement d'un dispositif (102) pour l'administration de liquide médical avec un corps principal (106) qui présente une zone de réception (108) et un entraînement, dans lequel la zone de réception (108) est appropriée afin de recevoir au moins par sections un corps creux contenant du liquide, et l'entraînement est approprié afin d'agir sur un corps creux reçu au moins par sections de telle manière qu'un liquide contenu dans le corps creux reçu au moins par sections soit refoulé, et un couvercle (104) relié de manière mobile au corps principal, couverclequi recouvre vers l'extérieur la zone de réception (108) dans une position fermée, dans lequel une unité de saisie blocable manuellement est prévue au niveau du couvercle (104) pour la saisie manuelle d'ordres au niveau du dispositif de commande de telle manière que dans la position fermée du couvercle (104), une saisie manuelle sur l'unité de saisie soit possible de l'extérieur au moins par sections, et un dispositif de verrouillage qui est approprié afin de verrouiller le couvercle (104) dans la position fermée, avec l'étape consistant à
détecter si le couvercle (104) est verrouillé dans la position fermée,
**caractérisé par** les étapes consistant à :
déterminer si l'unité de saisie est bloquée en cas de déverrouillage détecté du couvercle (104) fermé, et
bloquer l'unité de saisie si l'unité de saisie n'est pas bloquée en cas de déverrouillage détecté du couvercle (4).
